# EUROPEAN PATENT APPLICATION

(11) **EP 4 428 220 A1**
(43) Date of publication of application: **11.09.2024**
(21) Application number: 23172756.1
(22) Date of filing: 11.05.2023
(51) Int. Cl.: C12M 1/32, C12M 1/00, C12M 1/42

(54) **SYSTEM FOR IN VITRO ELECTROSTIMULATION OF CELLS**

(30) Priority: 07.03.2023 PL 44399123
(71) Applicant: Akademia Gorniczo-Hutnicza im. Stanislawa Staszica w Krakowie, 30-059 Krakow (PL)
(72) Inventor: Benko, Aleksandra, 31-637 Kraków (PL); Stencel, Marek, 32-085 Modlnica (PL); Pietryga, Krzysztof, 30-052 Kraków (PL)
(74) Representative: Balinska, Ewa

(57) **Abstract**

The subject of the invention relates to a system for *in vitro* electro stimulation of cells, comprising:
- plurality of independent, separate culture wells, wherein each separate culture well (1) is formed as a self-contained container in the shape of a geometric solid, having side walls (40) and a bottom, forming the base (20) of the well;
- a PCB (3) on which electrodes of opposite signs are mounted in pairs to supply an electrical charge, which electrodes are connected to a power supply unit, the PCB being provided with appropriately arranged openings (10) for visual observation of cultures in the culture wells and with a housing configured to house the culture wells and the PCB
the system being characteristic by independent separate culture wells (1) that are made of flexible biocompatible plastic material that can withstand autoclave sterilization temperatures and have a base (20) of the well that is shaped in a form of a flange element and made of translucent plastic, which base (20), together with the side walls (40) of the well (1) define a well chamber having electrodes (30) of opposite signs made of flexible material arranged in the base (20) of each well (1), wherein the individual culture wells (1) are arranged on the PCB (3) in correlation with the openings (10) and further the system can be equipped with a microcomputer (6) with a power supply unit.

## Description

The subject of the invention is a system for *in vitro* electrostimulation of cells. The electrostimulation system can be used in conducting cell and tissue culturing, in the diagnosis of genetic diseases, in the screening (screening tests) of new drugs, and in the development of new materials for biomedical applications.

### Description of the prior art

Standard cell cultures are conducted under static conditions. Cells are seeded on the bottom of a culture plate, flooded with culture medium (which provides them with nourishment) and incubated for a preset period of time in an incubator. The standard culture plate usually used, for example, a 24-well plate, consists of a bottom plate having predefined thickness, for example, about 20 mm, in which openings are arranged defining culture wells for receiving the test material or for seeding into them cells, and a top plate in the form of a lid configured to be placed on the bottom plate. During the course of the culturing, cell growth is monitored using an optical microscope, therefore it is important to have the bottom of the culture well that is translucent.

Since the cells are placed in a static environment, which does not fully reflect the interactions taking place in the organism, and in addition, the mechanism by which the material (biomaterial) itself reacts to external stimuli is also simplified, the results of such monitoring do not fully correspond to the results obtained in contact with tissues in the patient's body. Living organisms produce bioelectric currents, further mechanical stresses occur, and thus continuous remodeling and dynamic behavior of tissues take place. Therefore, it is important to carry out tests under more real-life conditions, that are more closely resembling the real ones, by providing appropriate external stimuli to cells.

For this reason, cultures conducted under dynamic conditions, which offer the possibility of better representation of real biological environment of cells, in which numerous stimuli (electrical, mechanical, etc.) take place, are becoming more and more important, wherein for this purpose, among others, modified plates for static culture are used in such a way that they induce appropriate stimuli in the culture. An increasingly common solution in this regard is electrostimulation or mechano-stimulation (i.e. mechanical stimulation), or a combination of them.

In known solutions, electrostimulation is carried out by use of a special cover plate, configured to be placed above or on a culture plate, in which cover plate electrodes are embedded, and the forcing of current flow is carried out using external power source. The cover plate, using lonOptix's C-dish solution as an example, consists of a PCB in which graphite electrodes or electrodes made of stainless steel are embedded. During cell culturing, the cover plate is placed on the culture plate in such a way that the electrodes are immersed in the culture medium. Both plates are then covered with a lid of the culture plate and placed in an incubator. The cover plate is then connected via a ribbon cable to an external power source and by application of voltage, the flow of current is forced between the two opposing electrodes and through the culture medium.

Electrostimulation parameters available in the device: alternating current stimulation, trapezoidal current waveform, frequency in the range of 0.01 to 99 Hz, pulse length in the range of 0.4 to 10 ms, voltage of ± 40 V. Example of alternating current stimulation with a trapezoidal waveform (two- or singlephase), amplitude of 1 V, frequency of 1 Hz, pulse length of 5 ms.

In the art, alternating current electrostimulation methods with other waveforms are also known, for example, rectangular or sinusoidal waveforms.

The use of direct current electrostimulation is also known.

This type of electrostimulation by passing an electric charge through the culture medium involves the risk of causing many negative electrochemical effects. In addition, the system does not allow analysis of the effect of electrostimulation through electro-conductive substrate - regardless of the parameters of the substrate, the electric charge flows primarily through the culture medium. Another disadvantage of the system is its low resistance to sterilization - the systems can only be sterilized by immersion in ethanol and UV sterilization. Assuming that the system is to be reusable, the inability to sterilize it in an autoclave significantly reduces the functionality of the system and increases the risk of biofilm build-up on the surface of the electrodes. The graphite (IonOptix) or stainless steel (Optics 11 Life) electrodes used in the system do not have optimal biocompatibility, and their rough surfaces promote adhesion of microorganisms. Another disadvantage of known systems is their low versatility - the plates cooperate only with dedicated signal sources, which have limited capabilities in terms of programming of voltage waveforms. Moreover, electrostimulation goes on by leading cable bundles into the culture incubator. This increases the risk of cell culture infection and lowers the ergonomics of work in the laboratory.

Another known system for electrostimulation is the system known as Biowire II^{™}, with limited market coverage, which serves to accelerate the maturation of stem cells into cardiomyocytes and to monitor their behavior in cell culture.

In scientific articles, reports on new developments for electrostimulation can be found. For example, in the dissertation by Lozano, R., et al., Electrical stimulation enhances the acetylcholine receptors available for neuromuscular junction formation. Acta Biomaterialia, 2016. 45: p. 328 - 339, the cells were seeded on the surface of an electrode, which was the bottom of the culture well. The electrode with the opposite sign was then immersed from above in the culture medium, in such a way that it was arranged parallel and opposite the cell culture. In this arrangement the flow of electrical charge is carried out by the culture medium, which is a significant limitation of this solution. Moreover, it is not universal, since when any different electroconductive scaffold is used, a completely new system to which the culture wells are permanently attached, must be prepared.

Systems in which electrostimulation is performed through the substrate are also known, described, for example, in the works: Meng, S., Z. Zhang, and M. Rouabhia, Accelerated osteoblast mineralization on a conductive substrate by multiple electrical stimulation. Journal of Bone and Mineral Metabolism, 2011. 29(5): p. 535 - 544 and Paun, I.A., et al., and Electrically stimulated osteogenesis on Ti-PPy/PLGA constructs prepared by laser-assisted processes. Materials Science and Engineering: C, 2015. 55: p. 61 - 69. In these dissertations, the electrically conductive substrate constitutes bottom of the culture well, and an electric current is passed through it by connecting two electrodes of opposite signs, powered by an external current source (DC current) to its both distal ends. In the second article cited, the electrostimulation system consists of an electroconductive substrate, which is the bottom of the culture well, to which two electrodes of opposite signs are connected.

Other known systems for *in vitro* culture are disclosed in patent publications US20200140802A1, US20210380950A1, US10544388B2, US10421955B2, and US7148059B1, which disclose, in addition to systems for electrostimulation, also solutions allowing additional or parallel mechanical stimulation.

In the solution presented in the US publication 2018/0072979 A1, a system for electrostimulation of cell cultures *in vitro* is provided with a unit for delivering an electrical signal simultaneously to multiple culture wells without disrupting the incubation process and causing infection. Each well is equipped with two electrodes of opposite signs that are placed at opposite ends of the well chamber. All the culture wells defining the well chambers along with the entire electrode system are placed in groups on sterilizable rigid glass plates, which in turn are placed in an outer plastic housing. The electrodes supply each culture well chamber with appropriately selected electrical signal waveforms from a signal source, wherein electrostimulation takes place through the culture medium, and thus it can induce adverse electrochemical phenomena. In addition, the presented system does not provide the possibility of independently selecting the regimes of electrostimulation in individual culture wells - in each of the wells in one unit, the forcing signal is always the same, and the actual parameters of the current flowing through the individual wells are impossible to control.

From patent publication No. US2017/0175103A1, a system for culturing of cells or tissues that are placed in culture wells of separate containers made of deformable, heat-resistant elastomer, for example, silicone rubber is known. The system includes a programmable controller, connected to a power source supplying a microprocessor-based control unit for programming the operation of the system in terms of electrostimulation and mechanical stimulation. The control unit feeds a programmable electrical signal to electrostimulation and a signal for mechanical stretching to the electromechanical stations. Each electromechanical station consists of culture wells, support base for stretching the culture wells, and electrodes for electrostimulation. The stations are mounted in the PCB (printed circuit board) plate, which is situated above them.

Separate culture wells mounted inside the electromechanical stations are subjected to synchronized, simultaneous, or separately conducted electrostimulation and mechanical stimulation (the latter is done by stretching the cell wells in a controlled manner). A pair of carbon electrodes can be immersed in each culture well, cooperating with it to conduct electrostimulation through the culture medium. PCB plate contains openings which allow for observation of the culture carried out in the culture wells of the containers. According to this solution, stimulation is carried out through the culture medium, which can cause the adverse effects described in relation to the publication presented above.

Despite the continuous development of cell differentiation technologies, there is a need for solutions that would allow combining the process of electrostimulation with the use of suitable cell substrates, that would be electroconductive and would have the appropriate bioactive and biofunctional properties to promote growth, differentiation and maturation of cells. Systems, discussed above, that are known in the art, do not provide suitable standards in which electrostimulation would take place through an electroconductive substrate with a complete separation from the culture medium. There is no known in the art solution that would also exhibit high versatility, be suitable for sterilization, and provide culture wells that allow monitoring the culture viability *in situ,* while it is being grown.

### Essence of the invention and beneficial effects

According to the invention, a solution has been developed that solves the problems of systems known in the art and provides an improved system that allows electrostimulation of cells through a culture substrate with complete separation from the culture medium, as well as the possibility of implementation of simultaneous, separate or synchronized mechanical stimulation, and further enables monitoring of cell viability *in vitro* during carrying out of the process. Electrostimulation through the culture medium or with charge flow directly through the seeded cells is also an option. Thanks to allowing electrostimulation through the culture substrate, the risk of adverse electrochemical effects due to electrostimulation by passing an electric charge through the culture medium is eliminated. Some examples of these negative effects are: water hydrolysis, deposition of morphotic media components on one of the electrodes due to the electrophoresis process, electrolysis of culture medium components, or, finally, a significant increase in the temperature prevailing in the culture well during stimulation.

Moreover, the proposed system for cell culture is suitable for sterilization of the culture wells together with the electrodes in an autoclave. By such sterilization, the risk of infection is reduced, which is essential especially for the long-term cell cultures.

The system according to the invention allows separate stimulation of each culture well with any desired signal. Furthermore, all components of the system, including each culture well, together with electrodes and a housing, are made entirely of biocompatible polymeric material. Thanks to its suitability for remote operation, the system allows to achieve high sterility in the incubator, and to provide high versatility of application.

The essence of the invention consists in development of a new system for electrostimulation of cells *in vitro* conditions, which can be further complemented with mechano-stimulation.

According to the invention, a new system for *in vitro* electrostimulation of cells contains:
- a plurality of independent and separate culture wells, made of flexible and biocompatible polymer, wherein each separate culture well is formed as a self-contained container in the shape of a geometric solid, in particular, in a cylinder-like shape, and has side walls and a bottom made of translucent material. The translucent bottom forms the base of the well, which, together with side walls, define a chamber of the well. Electrodes made of flexible material are arranged in the bottom, wherein two electrodes of opposite signs are placed opposite to each other on the bottom of each culture well. In preferred embodiment, the system may include a minimum of 8 independent separate culture wells, preferably made of PDMS (polydimethylsiloxane) material;
- a PCB (printed-circuit board) on which the separate culture wells are arranged in such a way that they are put on with their bottom on electrical connectors which are made, arranged and fixed on the PCB, in particular in the form of pins or plugs, and which preferably may be soldered into the PCB; wherein the PCB has imprinted a circuit of electrical connections, and has openings for observing the culture in the wells, respectively arranged at the locations in which the culture wells are placed, while on the opposite sides of these openings, there are places for installation of the said electrical connectors, two said electrical connectors per each opening;
- a housing in which the PCB is embedded, together with electrical connectors, and together with the culture wells, and which is made of a biocompatible polymer, for example, polylactide (PLA).

In preferred embodiment of the invention, an electrically conductive culture substrate can be placed at the bottom of the culture well.

Furthermore, in preferred embodiment of the invention, a voltage source, for example, in the form of an independent microcomputer and a digital-to-analog (D/A) converter, powered by an independent power unit, can also be placed in the housing.

In an alternative embodiment of the invention, the PCB may be equipped with an additional electrical power connector for connecting the electrostimulation system to an external function generator and/or power supply source, via a cable, preferably via a ribbon cable.

In the embodiment of the invention, a microcomputer is used to receive signal packets and generate, via a digital-to-analog (D/A) converter, a preset electrical signal in the respective selected culture wells, for electrostimulation of the cell culture. Preferably, each of culture wells can be subjected to electrical stimulation with a different, freely programmable (with respect to a shape, waveform, amplitude, period, phase, stimulation time, number of stimulation cycles) preset signal.

In the embodiment of the invention, signal packets are sent to the microcomputer wirelessly, for example, using a specially designed application located on a PC arranged outside the sterile room. The application allows selection of a shape of a current waveform, its period and a phase, and a stimulation time. It is also possible to implement the number of stimulation cycles.

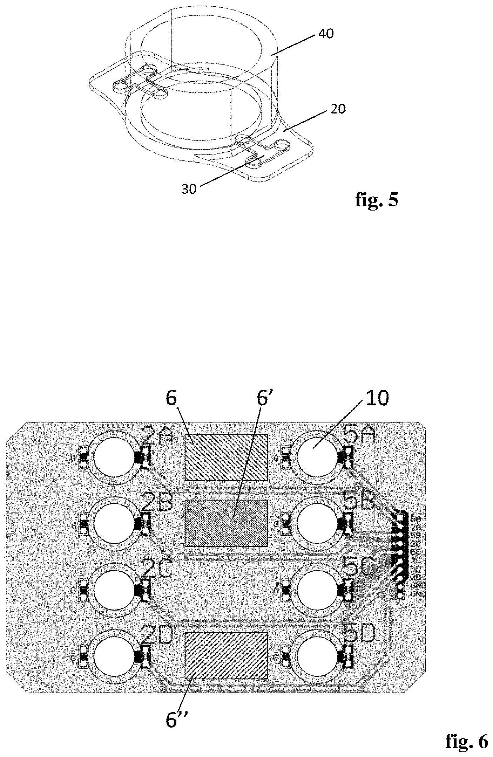

According to a recommended embodiment of the invention, the housing, advantageously 3D printed, houses PCB with attached electric connectors for the culture wells, in particular soldered pins for attaching separate, independent culture wells in such a way, that electrical contact is ensured at the boundary between the electrical connector and the flexible electrode embedded in the bottom of the respective culture well, in particular in the base of the respective culture well, and further, in a preferable embodiment, said housing additionally houses a microcomputer, a digital-to-analog converter and a power supply source, for example a power supplying battery (fig. 6).

### Short description of figures of the drawing

The subject of the invention in embodiments is shown in the drawing, in which fig. 1a shows schematically in perspective view the system for electrostimulation of cells in the first embodiment according to the invention, fig. 1b shows schematically in perspective view the system for electrostimulation of cells in the next embodiment according to the invention, fig. 2 shows schematically in perspective view the system for electrostimulation of cells in the next, different embodiment according to the invention, fig. 3 shows schematically in perspective view the system for electrostimulation of cells in yet another further embodiment according to the invention, fig. 4 shows schematically in perspective view the culture wells for the system for electrostimulation of cells in the first embodiment according to the invention, fig. 5 shows schematically in perspective view the culture wells for the system for electrostimulation of cells in the second embodiment according to the invention, fig. 6 shows schematically in top view the PCB for the system for electrostimulation of cells in an embodiment according to the invention.

### Description of embodiments

According to the invention, a new system for *in vitro* electrostimulation of cells, for conducting cell or tissue culturing is provided, which in the embodiment example consists of the following components:
- Separate independent culture wells 1 made of a flexible, biocompatible plastic material, for example, polydimethylsiloxane (PDMS) (e.g., Sylgard 184) or other silicones and siloxanes. On the well bottom forming the base 20 of each separate culture well 1, flexible electrodes 30 of opposite signs are arranged, which are made, for example, of PDMS plastic modified with carbon particles and/or carbon nanoparticles, in particular carbon nanotubes, while other known materials for electrodes may also be used. The culture wells 1 are shaped in a cylinder-like shape, each of them having a sidewall and a translucent bottom constituting the base 20 of the well, in which base flexible electrodes 30 are arranged, that is two electrodes of opposite signs per each culture well 1, which two electrodes 30 are arranged on both respectively opposite sides of the outline of the well bottom each. Preferably, the base 20 of the bottom is in the shape of a flanged element having two opposing protrusion elements, in each of which one electrode 30 is embedded, i.e. one in each protrusion element. The electrodes 30 in the preferred embodiment are of a near T-shape (near-letter T shape), wherein the base of the T-letter shape enters into the bottom of the culture well 1, and the arms of the T-letter both have openings at their free ends into each of which electrical connectors 5 enter, preferably pins 5 forming an electrical contact with the PCB 3. The pins 5 may be in the form of mandrels. The electrodes 30 are configured to be embedded in the base 20 of the wells, preferably in its flange element, and they may also be shaped in any other manner that will secure an electrical connection between the connectors 5 and the bottom of the culture well 1.

In the recommended embodiment the shape and the method of attachment of the electrodes 30 described above makes it possible to apply the process of electrostimulation through the culture substrate, whereby the cultured cells can be seeded onto an electrically conductive culture substrate placed at the bottom of the culture well 1. It is also possible to complement the system by including mechano-stimulation.
- A printed circuit board PCB 3 for arranging on it in a suitable configuration and fixing thereon a number of separate culture wells 1, the printed circuit board PCB have plurality of electrical connectors 5 for applying electrical signals to the respective cooperating electrodes 30 of the individual culture wells 1. Advantageously, the PCB can have a minimum of 8 electrical connectors 5, in particular in the form of pins or plugs. The electrical connectors 5 serve at the same time to attach the culture wells 1 on the PCB 3. In addition, the PCB 3 is designed in such a way that there are circular openings 10 in it, that are arranged in correlation with the culture wells 1 and at the locations in which the culture wells 1 are attached to the PCB 3. The openings 10 allow real-time observation of the cultured cells in the culture wells 1 through the translucent bottom of the well, using a microscope of suitable design.
- A housing 4 for the PCB 3 with attached on it (in spaces) culture wells 1 and for electrical and electronic components, the housing isolates all electrical components of the system from the negative effects of the external environment, including the high humidity and temperature of about 40°C prevailing in the incubator for culturing cells under *in vitro* conditions;

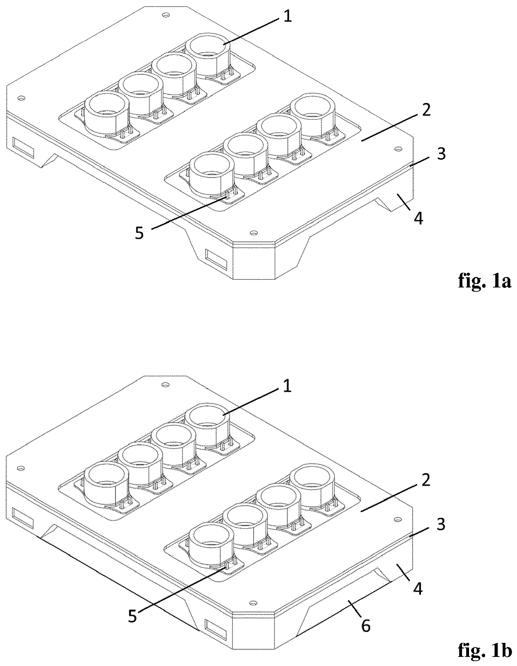

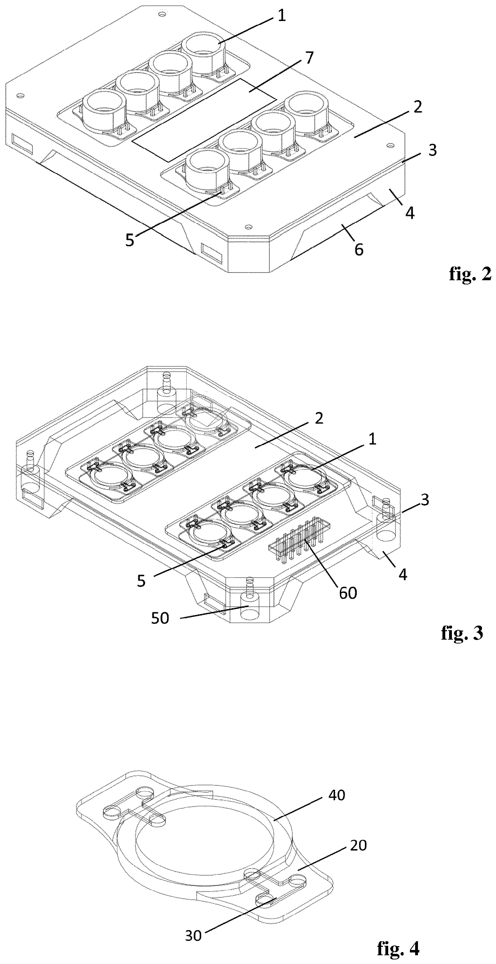

In embodiments of the invention the system for electrostimulation of cells *in vitro* conditions, shown in fig. 1b and 2 can be also equipped with:
- A microcomputer 6, a digital-to-analog (D/A) converter 6' and a power unit 6", preferably a power supplying battery or a USB power supply, which are housed inside the housing 4 of the PCB 3 and which make possible to generate of AC/DC signals *in situ,* inside the culture incubator (not shown), without the need for wiring or using of an external function generator;

In another embodiment, shown in fig. 3, a power connector 60 is located on the PCB 3, through which wiring can be connected to connect an external function generator and/or an external power source and/or an external computer or signal control microprocessor.

In preferred embodiment of the invention, the system for electrostimulation and, in particular, the microcomputer 6 include software for cooperation with a PC desktop application that allows remote communication with the system and the transmission of data packets (signals) wirelessly and directly to the microcomputer 6. The microcomputer 6 is equipped with software for receiving signals transmitted by means of the desktop application.

The power unit 6" can include a Li-Ion rechargeable battery (for example, with a capacity of 2000 - 6000 mAh) or, in an alternative embodiment, a USB Type-C power supply.

The system for electrostimulation of cells under *in vitro* conditions in the embodiment according to the invention shown in fig. 1a, 1b includes a PCB 3, whereby the PCB 3 has a printed electrical circuit with a circuit of connections scheme, an example electrical circuit diagram is shown in fig. 6. It includes the leads to the electrical connectors 5 for the corresponding number of independent culture wells 1, for example the fig. 6 shows independent leads for connectors 5 for up to eight (8) culture wells 1. The PCB 3 is provided with a suitable number of electrical connectors 5, that is preferably two connectors per opening, located on opposite sides of the openings 10 in the PCB each, and each of the connectors pair is configured to receive the culture well 1 through attachment embedded on each of them, as well as to ensure electrical connection of the respective well 1 on the PCB 3, whereby the PCB 3 may have any number of electrical leads to any number of culture wells, for example, 6, 12, 24 or any other number of electrical leads to the wells 1, in such a way that there are two opposite electrodes of opposite signs per culture well 1, each of which is electrically connected to the corresponding electrical connector 5. In the PCB 3 openings 10 are of a dimension adapted to the diameter of the culture wells 1 and are provided at the locations in which the culture wells 1 are located. The openings 10 enable observation and monitoring of the growth of cells in the culture wells 1 in a manner analogous to that in standard culture plates.

In each of two opposite sides of each opening 10, respectively, one of two electrical connectors 5, in particular in the form of pair of pins 5, is positioned and fixed, advantageously by soldering, so that there are two pairs of electrical connectors 5 on opposite sides of each opening 10 in the PCB 3. The electrical connectors 5, in particular the pins 5, are electrical leads for providing an electrical connection between the PCB 3 and the culture wells 1, and particularly the electrodes 30 of the culture wells 1. The pins 5 may be in the form of mandrels or plugs, or in any other known form allowing an electrical signal to be supplied to the electrodes 30 located in the bottom of the culture wells 1.

The culture wells 1, shown by example in fig. 4 and fig. 5 in cylindrical shape with a flange-shaped base 20 of the well, are made of a flexible, biocompatible material, for example PDMS-type (polydimethylsiloxane-type) material, which is a transparent material, for example such as Sylgard 184 material. In the recommended manufacturing example, they have dimensions similar to the culture wells in a conventional cell culture plate, i.e. radius of 3.1 to 17.5 mm. On the bottom, in the form of the base 20 of the well, of each culture well 1, there are arranged flexible electrodes 30, two per each well and each of them is located on the opposite sides of the base 20 of the well, made of flexible material, for example made of an electrically conductive PDMS plastic material modified with carbon particles and/or nanoparticles, in preferable embodiment modified with carbon nanotubes. Due to the flexibility of the culture wells 1 it is possible to use mechano-stimulation (not shown) by means of stepper motors.

The culture wells 1 are reusable, they can be easily dismounted from the PCB 3 and reassembled (mounted again) on it and connected to the electrical connectors 5, they are suitable for cleaning and sterilization using an autoclave. The wells 1 are made of a material that can withstand the high temperature prevailing in the autoclave in the range above 120°C, advantageously in the range above 140°C, in particular in the range from 140 to 200°C.

The housing 4 of the PCB 3 can be made by 3D printing techniques (e.g., Fused Deposition Modeling (FDM), or Stereolithography (SLA)) and the PCB 3 is placed thereon and covered by a cover or lid 2 from above. The housing 4 is, in the embodiment, connected to the lid 2 by screws 50, advantageously four screws, and it is sealed by means of a U-seal made of polydimethylsiloxane (PDMS). The whole unit can optionally be additionally covered with a top cap (not shown) for minimizing evaporation of the medium.

In the recommended embodiment, the microcomputer 6 (see fig. 2) is located in a lower part of the housing 4 (fig. 6) and may be of a type of a raspberry pi zero 2W or equivalent type computer or another one, operating in a Linux environment, and it is adapted to receive signal packets and to generate a preset electrical signal via the digital-to-analog converter 6', which signal is fed via the electrical circuit and electrical connectors 5 on the PCB 3 to the corresponding electrodes 30 of the respective or selected culture wells 1. The signal packets are sent to the microcomputer 6 wirelessly by any known way, in particular by means of a computer application which is located on a cooperating PC type computer located outside the sterile room, in which the system, according to the invention, is located. This application gives the possibility to choose the shape of the current waveform of the signal, its period and a phase, and the stimulation duration time. It is also possible to preset the number of stimulation cycles. The microcomputer 6 can be powered by a battery, for example, a lithium-ion battery, which is included in the system and located in the lower part 4 of the housing 2,4. The microcomputer 6 is responsible for receiving these signal packets and generating, via the D/A 60, a preset electrical signal in the individual culture wells, the signal being transmitted wirelessly.

In addition, or alternatively in the embodiment, the PCB 3 is equipped with a power connector 60 which allows connection to an external function generator, via a cable, for example a ribbon cable.

The system for electrostimulation according to the invention is adapted to be placed in an incubator (not shown, known in the art) for cell culturing, wherein the cells designated to be cultured under electrostimulation conditions can be preferably seeded onto an electrically conductive substrate at the bottom of the culture wells 1, which is provided with two opposite electrodes 30, which are placed on opposite sides of the well 1, and each of which is connected to an electrical connector 5 on the PCB 3.

In a further embodiment of the invention, additionally or alternatively, the PCB 3 is preferably provided with an LCD display 7 connected to it, which shows the status of the system, for example, the signals generated in the individual culture wells, the culture number and the like.

According to the invention, at least two types of culture wells 1 are used in the embodiments, exemplified in figs. 4 and 5, and figs. 2 and 3, which can be placed and fixed and connected via electrical connectors 5 on a PCB 3, preferably via pins 5, depending on the type of a culture. In an embodiment of the invention (fig. 4) the culture well 1 has a minimum volume, which, in the case of a cylindrical shape of the well, is determined by the standardized diameter of the culture well and the minimum height of its side wall 40, so that a minimum required volume of the culture medium is ensured.

In a further embodiment of the invention (fig.5), the culture well 1 has a larger volume, which in the case of the cylindrical shape of the well is determined by the normalized diameter of the culture well and the height of its side wall that is respectively increased to the corresponding larger dimensions to ensure the required larger volume of the culture medium. Embodiments of an electrostimulation system with culture wells of minimum height are shown in fig. 3, whereby different dimensions of volume of the culture wells can be used in each of the above-described embodiment of the system according to the invention, depending on the needs.

The culture wells 1 can be sized to match the standard dimensions of the wells in the culture plate (radius from 3.1 to 17.5 mm), and the standard dimension is that of the wells in a 24-well plate (radius of about 7.8 mm).

The housing can be made of a biocompatible polymer, such as, for example, polylactide (PLA), and can be, for example, closed by means of screws, for example, four screws, and sealed with a plastic seal, for example, polydimethylsiloxane (PDMS).

The system according to the invention allows, for example, the simultaneous generation of 8 independent signals and can be additionally equipped with a power connector 60 that ensures connection of an external function generator, thus providing a certain versatility. A schematic diagram and an embodiment of the proposed PCB 3 is shown in fig. 6.

## Claims

1. The system for *in vitro* electrostimulation of cells, comprising:
- plurality of independent, separate culture wells, wherein each separate culture well is formed as a self-contained container in the shape of a geometric solid, having side walls and a bottom, forming the base of the well;
- a PCB on which electrodes of opposite signs are mounted in pairs to supply an electrical charge, which electrodes are connected to a power supply unit, the PCB being provided with a printed circuit in the form of an electrical connection system for supplying an electrical signal, and it has appropriately arranged openings for visual observation of cultures in the culture wells, preferably by means of a microscope;
- a housing configured to house the culture wells and the PCB and suitable for insertion into an incubator for cells culturing;
**characterized in that**
the independent separate culture wells (1) are made of flexible biocompatible plastic material that can withstand autoclave sterilization temperatures, preferably above 120°C, especially above 140°C, and have a base (20) of the well that is shaped in a form of a flange element and made of translucent plastic, which base (20), together with the side walls (40) of the well (1) define a well chamber having electrodes (30) of opposite signs made of flexible material arranged in the base (20) of each well (1), wherein the individual culture wells (1) are arranged on the PCB (3) in such a way, that they are put on at their bottom sides on electrical connectors (5) which are made, arranged and fixed on the PCB (3) in pairs, in such a manner that each connector of the pair is arranged on opposite sides of the corresponding opening (10) for observation, wherein each electrical connector (5) is electrically connected to the corresponding electrode (30) of a pair of electrodes (30) that are arranged on opposite sides of the base (20) of each culture well (1), and wherein the PCB (3) together with the culture wells (1) arranged thereon is positioned in the housing (4).

2. The system according to claim 1, **characterized in that** it is a system for electrostimulation through a substrate, whereby an electrically conductive culture substrate is placed on the bottom of the culture wells (1) for seeding the culture cells.

3. The system according to one of claims 1 - 2, **characterized in that** the electrical connectors (5) are made in the form of pins or plugs and they are attached, preferably they are soldered, in the PCB (3) on opposite sides of the respective openings (10) for observation, respectively two connectors (5) per each opening (10).

4. The system according to one of claims 1 - 3, **characterized in that** the culture wells (1) have a cylindrical-like shape and are positioned and fixed on the PBC (3) at the attachment points of the electrical connectors (5) in correlation with the openings (10) that is in the way corresponding to openings (10), wherein the base (20) of each culture well (1) is in the shape of a flange element with two opposite projecting elements, in each of which one an electrode (30) is embedded.

5. The system according to claim 4, **characterized in that** the electrodes (30) are of a T-shape, wherein a base of the letter T enters into the bottom of the culture well (1), while arms of the letter T have openings at their opposite free ends each, into which openings, electrical connectors (5), preferably pins (5), extend, thus forming an electrical contact with the PCB (3).

6. The system according to any one of claims 1-5, **characterized in that** the electrodes (30) of opposite signs, arranged two per each culture well (1), each on opposite sides of the well base (20), are made of a flexible material, advantageously they are made of an electrically conductive PDMS plastic material modified with carbon particles and/or carbon nanoparticles, especially and preferably modified with carbon nanotubes.

7. The system according to any one of claims 1-6, **characterized in that** it comprises a microcomputer (6) with a digital-to-analog (D/A) converter (6') and with a power unit (6"), preferably a power supplying battery or a USB power supply for generating AC/DC signals *in situ,* which are arranged in the housing (4) together with the PCB (3) and the culture wells (1).

8. The system according to any one of claims 1-7, **characterized in that** it comprises the microcomputer (6), which is equipped with software for wirelessly receiving signal packets and generating via the digital-to-analog (D/A) converter (6') a preset electrical signal in respective selected culture wells (1) for electrostimulation of cell culture, wherein preferably the preset signal for electrostimulation is freely programmable for each of culture wells (1) in terms of a shape, a waveform, an amplitude, a period, a phase, stimulation duration time and a number of stimulation cycles.

9. The system according to claim 8, **characterized in that** the microcomputer (6) is equipped with software for wireless reception of signal packets via a desktop application for a PC.

10. The system according to any one of the claims 1-9, **characterized in that** the PCB (3) is equipped with an additional electrical power connector (60) for connection via a cable, preferably a ribbon cable, to an external function generator and/or power source.

11. The system according to any one of claims 1 - 10, **characterized in that** the culture wells (1) are shaped as cylindrical, wherein the culture wells (1) are made of a biocompatible material of polydimethylsiloxane (PDMS) or other silicone or siloxane, while the housing (4) is made of a biocompatible polymer such as polylactide (PLA), preferably by means of 3D printing technique.
